(19) Europäisches Patentamt
European Patent Office
Office européen des brevets

(11) EP 1 491 180 A1

(12) EUROPEAN PATENT APPLICATION

(43) Date of publication:
29.12.2004 Bulletin 2004/53

(51) Int Cl.7: A61K 7/075

(21) Application number: 04014468.5

(22) Date of filing: 21.06.2004

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(30) Priority: 20.06.2003 JP 2003176578

(71) Applicant: Kao Corporation
Tokyo 103-8210 (JP)

(72) Inventors:
- Sakai, Hirokazu
Tokyo 131-8501 (JP)
- Okamoto, Yoshimasa
Tokyo 131-8501 (JP)

(74) Representative: HOFFMANN - EITLE
Patent- und Rechtsanwälte
Arabellastrasse 4
81925 München (DE)

# (54) Preparation process of a hair cosmetic composition

(57) Provided are a preparation process for a hair cosmetic composition having the step of emulsifying an aqueous composition having (A) an amphipathic amide lipid, (B) a quaternary ammonium salt (b-1) or tertiary amine type compound (B-2) or salt thereof, and (C) a higher alcohol (c) :

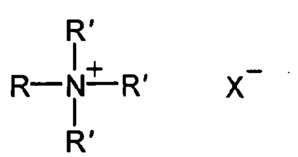

(b-1)

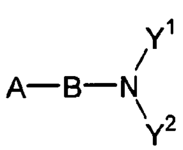

(b-2)

R"— OH (c)

(wherein, R and R" represents $C_{12-28}$ aliphatic hydrocarbon, R' represents $C_{1-3}$ alkyl, $X^-$ represents anion, A represents H or amide group having 12 to 24 carbon atoms in total, N-(hydrocarbon carbamoyl), acyloxy or hydrocarbon oxy, B represents $C_{1-22}$ divalent hydrocarbon, and $Y^1$ and $Y^2$ represents $C_{1-4}$ alkyl) at a temperature equal to or greater than the melting point of Component (C) but less than the phase transition temperature of a composition composed of Components (B) and (C), each in an amount equal to the content of the intended hair cosmetic composition, and balance water; and a hair cosmetic composition thus obtained.

The hair cosmetic composition obtained by the process described above provides benefits such as excellent lubrication and flexibility to the hair during from application to washing-away (rinsing), provides resilience, body and effect-remaining feel for the hair after drying and at the same time, has excellent effects for preventing hair damage and has excellent emulsion stability.

EP 1 491 180 A1

## Description

### FIELD OF THE INVENTION

The present invention relates to a process for preparing a hair cosmetic composition capable of imparting benefits such as a pleasant feel to hair and preventing hair damage.

### BACKGROUND OF THE INVENTION

Hair cosmetic compositions such as rinse, conditioner, treatment and the like have been used for the purposes of preventing hair entanglement and improving the hair feel after shampooing. A cationic surfactant and a higher alcohol are added to such hair cosmetic compositions, but their effects for improving the hair feel such as flexibility and moisturized feel are insufficient. In recent days, consumers tend to give frequent chemical stimulation to their hair such as permanent waving, hair coloring and bleaching and their hair is severely damaged with a partial component or structural loss. The conventional hair cosmetic compositions cannot overcome the problems such as friction between hair strands upon rinsing or problems relating to the protection or repair of the hair.

As a method for providing a hair cosmetic composition capable of improving the lubrication and flexibility of the hair during from application to rinsing and having improved emulsion stability, disclosed is a process for preparing the hair cosmetic composition by emulsifying at a temperature lower than the phase transition temperature of the hair cosmetic composition to be prepared but equal to or greater than the melting point of the higher alcohol to be incorporated in the composition (Japanese Patent Laid Open No. 2002-29937). This process however cannot bring about sufficient effects for improving the lubricated feeling of the hair from application to rinsing, and for protecting or repairing the hair.

It is a common practice to protect or repair the damaged hair by making up for a component or structure, or analogue thereof lost by damage. A process of using an amphipathic amide lipid such as a sphingolipid and the like or protein derivative as a protecting base has been employed widely. The amphipathic amide lipid has a high melting point and crystallizes readily, so that a sufficient amount of it cannot be added. In the case of a rinse-type hair cosmetic composition, it is mostly washed away upon rinsing and therefore does not bring about sufficient effects.

### SUMMARY OF THE INVENTION

In one aspect of the present .invention, there is thus provided a process for preparinga hair cosmetic composition containing the following components (A), (B) and (C):

(A) an amphipathic amide lipid,
(B) a quaternary ammonium salt represented by the following formula (b-1):

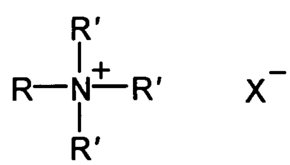

(b-1)

(wherein, R represents a $C_{12-28}$ aliphatic hydrocarbon group, R' represents a $C_{1-3}$ alkyl group and $X^-$ represents an anion), or a tertiary amine type compound represented by the following formula (b-2):

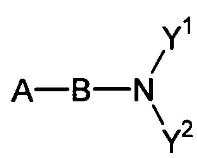

(b-2)

(wherein, A represents a hydrogen atom or a linear or branched, and saturated or unsaturated amide, N-hydrocarbon carbamoyl group, acyloxy group or hydrocarbon oxy group having 12 to 24 carbon atoms in total, B represents a linear or branched, saturated or unsaturated divalent $C_{1-22}$ hydrocarbon group, and $Y^1$ and $Y^2$ each independently represents a $C_{1-4}$ alkyl group) or salt thereof, and

(C) a higher alcohol represented by the following formula (c):

$$R''\text{-}OH \qquad (c)$$

(wherein, R" represents an aliphatic $C_{12\text{-}28}$ hydrocarbon group), the process having the step of emulsifying an aqueous composition containing Components (A), (B) and (C) at a temperature equal to or greater than the melting point of Component (C) but below the phase transition temperature of a three-component composition composed of Component (B) and Component (C), each of the Components added in amounts equal to the contents thereof in the intended hair cosmetic composition, respectively, and the balance water.

In another aspect of the present invention, there is also provided a hair cosmetic composition containing the above-described Components (A), (B) and (C) and which is made by emulsifying an aqueous composition containing Components (A), (B) and (C) at a temperature equal to or greater than the melting point of Component (C) but below the phase transition temperature of a three-component composition composed of Component (B) and Component (C), each of the Components added in amounts equal to the contents thereof in the intended hair cosmetic composition, respectively, and the balance water.

## DETAILED DESCRIPTION OF THE INVENTION

The present invention relates to a process for preparing a hair cosmetic composition capable of for example imparting excellent lubrication and flexibility to the hair during from application to washing (rinsing), giving resilience, body and effect-remaining feel to the hair after drying, being effective for preventing hair damage and moreover, having excellent emulsion stability.

The present inventors have found that a hair cosmetic composition capable of satisfying the above-described demand can be obtained by, upon preparation of a hair cosmetic composition containing an amphipathic amide lipid, a quaternary ammonium salt and a higher alcohol, emulsifying them at a temperature equal to or greater than the melting point of the higher alcohol but below the phase transition temperature of a three-component composition composed of the quaternary ammonium salt and the higher alcohol, in amounts equal to the contents thereof in the intended hair cosmetic composition, respectively, and the balance water. Because, the amphipathic amide lipid serving as a protecting base is stably dispersed in an oil layer of a liquid crystal lamellar layer, even if the composition is a rinse type and is washed away from the hair upon treatment, it can be effectively left on the hair and penetrated into the hair.

The amphipathic amide lipid as Component (A) preferably has 1 or 2 amide groups; preferably has, as a carbon chain bonded to the carbonyl group of the amide group, a $C_{5\text{-}60}$ alkyl or alkylene group which may be substituted with a hydroxy group and may contain an ester bond in its main chain; and preferably contains 1 to 5 hydroxy or $C_{1\text{-}30}$ alkoxy groups in total. The following compounds (A-1) to (A-4) are specific examples of the amphipathic amide lipid.

(A-1) Diamide compounds represented by formula (1):

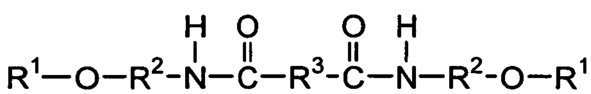

(1)

wherein, $R^1$ represents a linear or branched $C_{1\text{-}12}$ hydrocarbon group which may be substituted with a hydroxy group (s) and/or alkoxy group(s), $R^2$ represents a linear or branched divalent $C_{1\text{-}5}$ hydrocarbon group and $R^3$ represents a linear or branched divalent $C_{1\text{-}22}$ hydrocarbon group.

As $R^1$ in formula (1), linear or branched $C_{1\text{-}12}$ alkyl groups which may be substituted with 1 to 3 groups selected from a hydroxy group and $C_{1\text{-}6}$ alkoxy groups are preferred. Of these, the unsubstituted $C_{1\text{-}12}$ alkyl groups and $C_{2\text{-}12}$ alkyl groups substituted with 1 to 2 hydroxy groups and one $C_{1\text{-}6}$ alkoxy group or with one hydroxy group and one $C_{1\text{-}6}$ alkoxy group are more preferred. Specific examples include methyl, ethyl, propyl, butyl, hexyl, dodecyl, 2-methylpropyl, 2-ethylhexyl, 2-hydroxyethyl, 9-hydroxynonyl, 2,3-dihydroxypropyl, 2-methoxyethyl, 2-hydroxy-3-methoxypropyl and 9-methoxynonyl groups, of which 2-hydroxyethyl, methyl, dodecyl and 2-methoxyethyl groups are preferred.

As $R^2$ in formula (1), linear or branched $C_{2\text{-}5}$ alkylene groups are preferred, and linear or branched $C_{2\text{-}3}$ alkylene groups are more preferred. Specific examples include ethylene, trimethylene, tetramethylene, pentamethylene, 1-methylethylene, 2-methylethylene, 1-methyltrimethylene, 2-methyltrimethylene, 1,1-dimethylethylene and 2-ethyltrimethylene groups. Of these, ethylene and trimethylene groups are preferred.

As $R^3$ in formula (1), linear or branched divalent $C_{2\text{-}22}$ hydrocarbon groups are preferred, and linear or branched $C_{11\text{-}22}$ alkylene groups and alkenylene groups having 1 to 4 double bonds are more preferred. Specific

examples include ethylene, trimethylene, tetramethylene, hexamethylene, heptamethylene, octamethylene, decamethylene, undecamethylene, dodecamethylene, tridecamethylene, tetradecamethylene, hexadecamethylene, octadecamethylene, 1-methylethylene, 2-ethyltrimethylene, 1-methylheptamethylene, 2-methylheptamethylene, 1-butylhexamethylene, 2-methyl-5-ethylheptamethylene, 2,3,6-trimethylheptamethylene, 6-ethyldecamethylene, 7-methyltetradecamethylene, 7-ethylhexadecamethylene, 7,12-dimethyloctadecamethylene, 8,11-dimethyloctadecamethylene, 7,10-dimethyl-7-ethylhexadecamethylene, 1-octadecylethylene, ethenylene, 1-octadecenylethylene, 7,11-octadecadienylene, 7-ethenyl-9-hexadecamethylene, 7,12-dimethyl-7,11-octadecadienylene and 8,11-dimethyl-7,11-octadecadienylene groups. Of these, 7,12-dimethyloctadecamethylene, 7,12-dimethyl-7,11-octadecadienylene, octadecamethylene, undecamethylene and tridecamethylene groups are preferred.

Preferred diamide compounds (1) are compounds having the above-described preferred groups as $R^1$, $R^2$ and $R^3$, respectively. Specific examples are the following compounds:

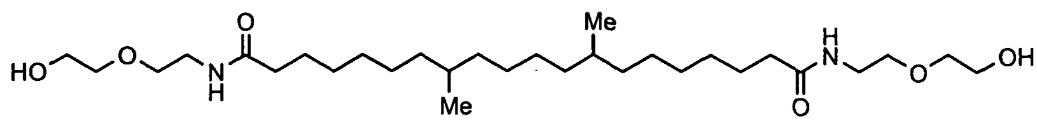

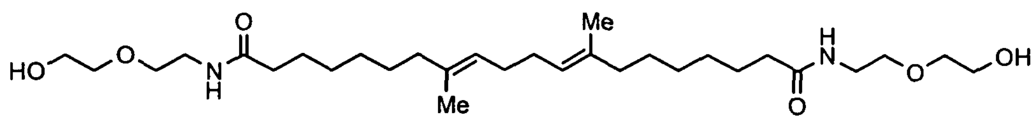

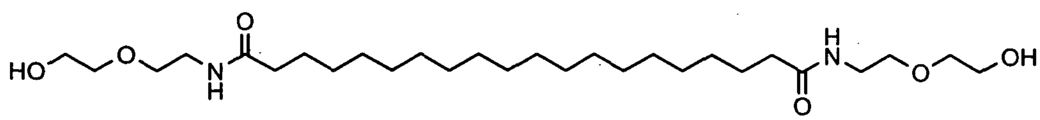

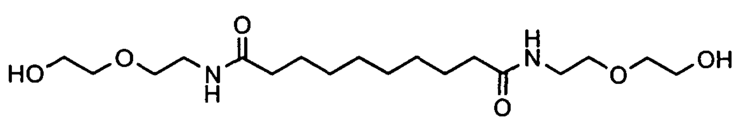

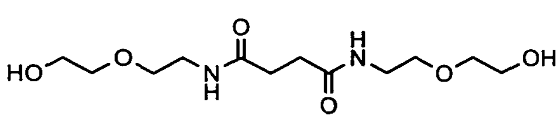

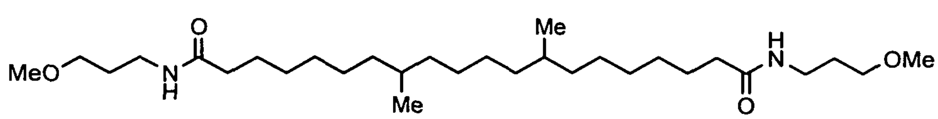

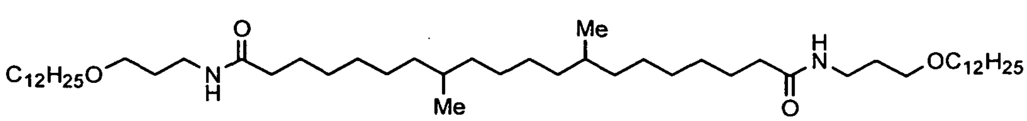

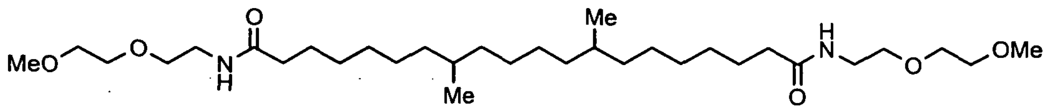

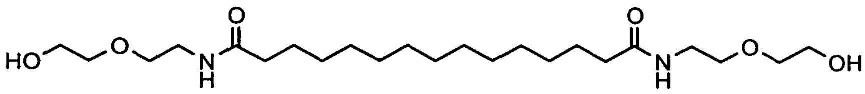

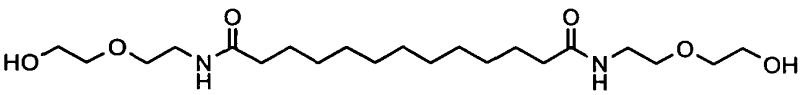

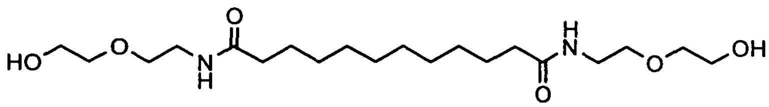

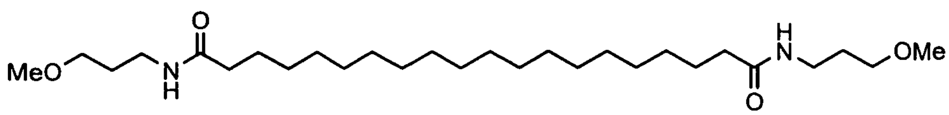

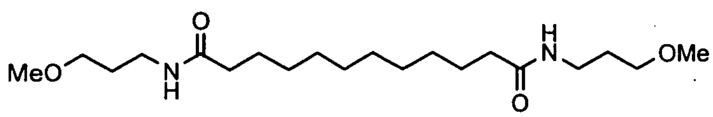

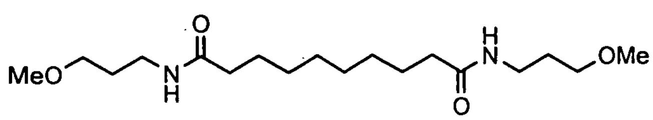

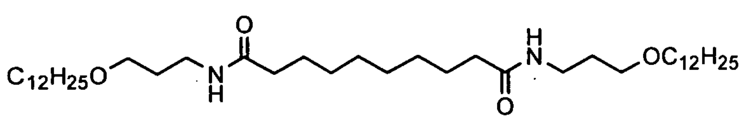

(A-2) Ceramides represented by the following formula (2):

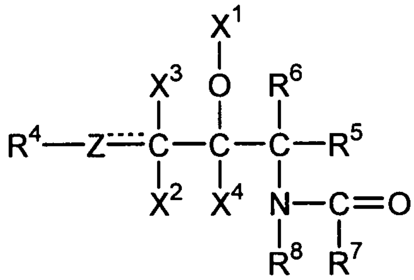

(2)

wherein, $R^4$ represents a linear, branched or cyclic, saturated or unsaturated $C_{4-30}$ hydrocarbon group which may be substituted with hydroxy, oxo or amino group(s), Z represents a methylene group, a methine group or an oxygen atom, a broken line represents the presence or absence of a π bond, $X^1$ represents a hydrogen atom, an acetyl group or a glyceryl group, or, forms an oxo group together with the adjacent oxygen atom, $X^2$, $X^3$ and $X^4$ each independently represent a hydrogen atom, a hydroxy group or an acetoxy group (with the proviso that when Z represents a methine group, either $X^2$ or $X^3$ represents a hydrogen atom and the other does not exist, and when -O-$X^1$ represents an oxo group, $X^4$ does not exist), $R^5$ and $R^6$ each independently represents a hydrogen atom, a hydroxy group, a hydroxymethyl group or an acetoxymethyl group, $R^7$ represents a linear, branched or cyclic, saturated $C_{5-35}$ hydrocarbon group which may be substituted with a hydroxy or amino group, or the saturated $C_{5-35}$ hydrocarbon group in which a linear, branched or cyclic, saturated or unsaturated $C_{8-22}$ fatty acid which may be substituted with hydroxy group(s) is ester-bonded at the ω-position of the hydrocarbon group, and $R^8$ represents a hydrogen atom or a linear or branched, saturated or unsaturated hydrocarbon group which may have substituent(s) selected from a hydroxy group, hydroxyalkoxy groups, alkoxy groups and an acetoxy group, and has 1 to 8 carbon atoms in total.

As $R^4$ in formula (2), linear, branched or cyclic, saturated or unsaturated $C_{7-22}$ hydrocarbon groups which may be substituted with hydroxy group(s) are preferred. As $X^1$, a hydrogen atom and a glyceryl group are preferred. It is preferred that at most one of $X^2$, $X^3$, and $X^4$ represents a hydroxy group and the others represent a hydrogen atom. It is preferred that one of $R^5$ and $R^6$ represents a hydrogen atom or a hydroxymethyl group and the other represents a hydrogen atom. In $R^7$, preferred examples of the fatty acid which may be ester-bonded or amide-bonded to the saturated hydrocarbon group at the ω-position thereof include isostearic acid, 12-hydroxystearic acid and linoleic acid. As $R^8$, a hydrogen atom and hydrocarbon groups having 1 to 8 carbon atoms in total which may be substituted with 1 to 3 substituents selected from a hydroxy group, a hydroxyalkoxy group and an alkoxy group are preferred.

As ceramide (2), preferred are the following compounds (2a) and (2b).

(A-2a) Natural ceramides or natural type ceramides represented by formula (2a), and derivatives thereof (which will hereinafter be called "natural type ceramides")

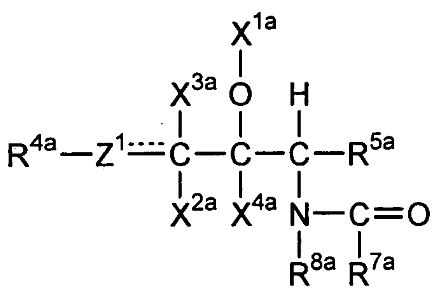

(2a)

wherein, $R^{4a}$ represents a linear, branched or cyclic, saturated or unsaturated $C_{7-19}$ hydrocarbon group which may be substituted with a hydroxy group, $Z^1$ represents a methylene or methine group, a broken line represents the presence or absence of a π bond, $X^{1a}$ represents a hydrogen atom or, forms an oxo group together with the adjacent oxygen atom, $X^{2a}$, $X^{3a}$ and $X^{4a}$ each independently represent a hydrogen atom, a hydroxy group or an acetoxy group (with the proviso that when $Z^1$ represents a methine group, one of $X^{2a}$ and $X^{3a}$ represents a hydrogen atom and the other does not exist, and when -O-$X^{1a}$ represents an oxo group, $X^{4a}$ does not exist), $R^{5a}$ represents a hydroxymethyl group or an acetoxymethyl group, $R^{7a}$ represents a linear, branched or cyclic, saturated $C_{5-30}$ hydrocarbon group which may be substituted with hydroxy group(s), or the saturated $C_{5-30}$ hydrocarbon group in which a linear or branched, saturated or unsaturated $C_{8-22}$ fatty acid which may be substituted with hydroxy group(s) is ester-bonded at the ω-position of the hydrocarbon group, and $R^{8a}$ represents a hydrogen atom or a $C_{1-4}$ alkyl group.

Preferred are compounds in which $R^{4a}$ is a linear $C_{7-19}$, more preferably $C_{13-15}$, alkyl group, $Z^1$ is a methine

group in which one of $X^{2a}$ and $X^{3a}$ is a hydrogen atom, and $R^{7a}$ is a linear $C_{9-27}$ alkyl group which may be substituted with hydroxy group(s). In addition, $X^{1a}$ preferably represents a hydrogen atom, or forms an oxo group together with an oxygen atom. Preferred examples of $R^{7a}$ include a tricosyl group, a 1-hydroxypentadecyl group, a 1-hydroxytricosyl group, a heptadecyl group, a 1-hydroxyundecyl group and a nonacosyl group having a linoleic acid ester-bonded at the ω-position thereof.

Specific examples of the natural type ceramides include Ceramide Types 1 to 7 having the structures described below which are obtained by amidation of sphingosine, dihydrosphingosine, phytosphingosine or sphingadienine (for example, FIG. 2 of J. Lipid Res., 24: 759(1983), and pig and human ceramides as described in FIG. 4 of J. Lipid Res., **35**: 2069(1994)).

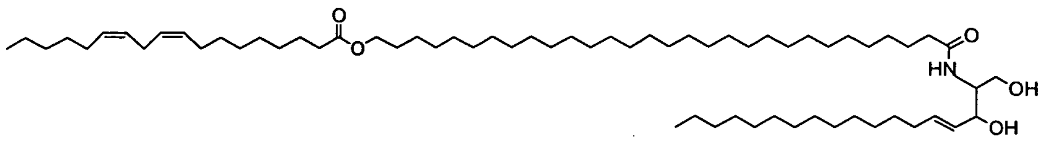

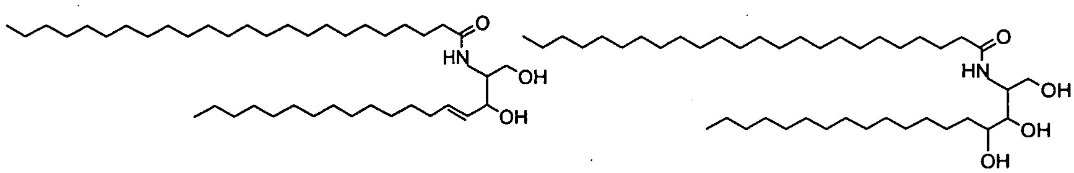

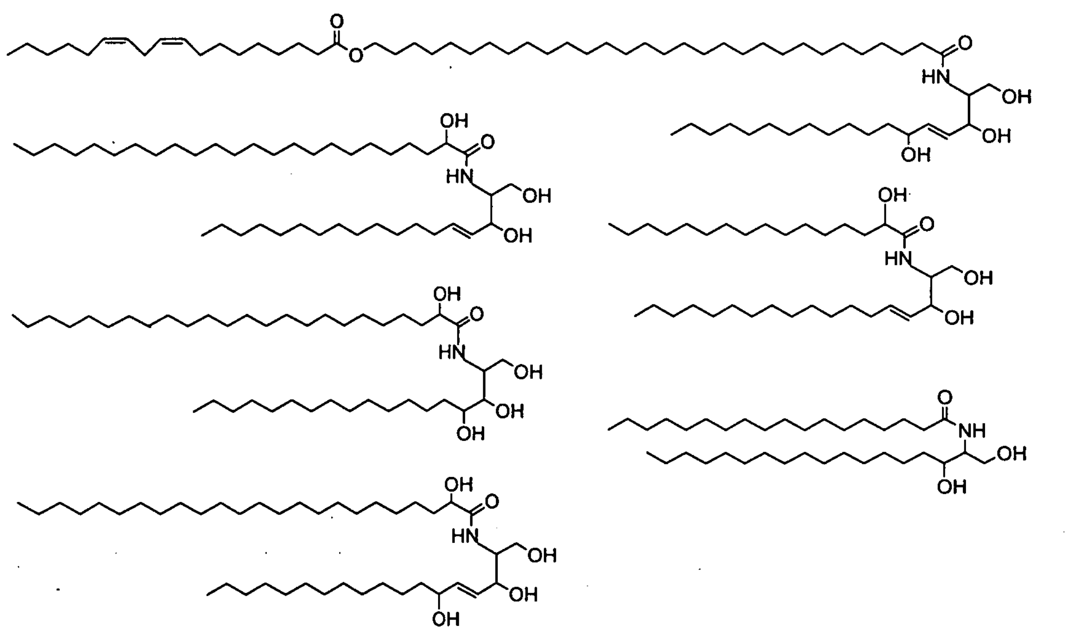

Examples also include N-alkyl derivatives (e.g., N-methyl derivatives) of the above-described ceramides. They may be either a natural extract or synthesized product. Commercially available ones can also be used.

(A-2b) Pseudo type ceramides represented by the following formula (2b):

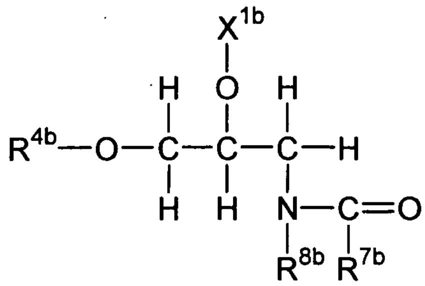

(2b)

wherein, $R^{4b}$ represents a linear, branched or cyclic, saturated or unsaturated $C_{10-22}$ hydrocarbon group which may be substituted with hydroxy group(s), $X^{1b}$ represents a hydrogen atom, an acetyl group or a glyceryl group, $R^{7b}$ represents a linear, branched or cyclic, saturated or unsaturated $C_{5-22}$ hydrocarbon group which may be substituted with hydroxy or amino group(s), or the hydrocarbon group in which a linear or branched, saturated or unsaturated $C_{8-22}$ fatty acid which may be substituted with hydroxy group(s) is ester-bonded at the ω-position of the hydrocarbon group, and $R^{8b}$ represents a hydrogen atom or an alkyl group having 1 to 8 carbon atoms in total which may be substituted with hydroxy group(s), hydroxyalkoxy group(s), alkoxy group(s) or acetoxy group(s).

Preferred as $R^{7b}$ are a nonyl group, a tridecyl group, a pentadecyl group, an undecyl group having linoleic acid ester-bonded at the ω-position thereof, a pentadecyl group having linoleic acid ester-bonded at the ω-position thereof, a pentadecyl group having 12-hydroxystearic acid ester-bonded at the ω-position thereof, and an undecyl group having methyl-branched isostearic acid amide-bonded at the ω-position thereof. The hydroxyalkoxy or alkoxy groups for $R^{8b}$ are preferred to have 1 to 8 carbon atoms.

As the pseudo type ceramides (2b), those having as $R^{4b}$ a hexadecyl group, as $X^{1b}$ a hydrogen atom, as $R^{7b}$ a pentadecyl group, and as $R^{8b}$ a hydroxyethyl group; those having as $R^{4b}$ a hexadecyl group, as $X^{1b}$ a hydrogen atom, as $R^{7b}$ a nonyl group, and as $R^{8b}$ a hydroxyethyl group; or those having as $R^{4b}$ a hexadecyl group, as $X^{1b}$ a glyceryl group, as $R^{7b}$ a tridecyl group, and as $R^{8b}$ a 3-methoxypropyl group are preferred, with those having as $R^{4b}$ a hexadecyl group, as $X^{1b}$ a hydrogen atom, as $R^{7b}$ a pentadecyl group, and as $R^{8b}$ a hydroxyethyl group being more preferred. Specifically preferred examples include those represented by the following formulas:

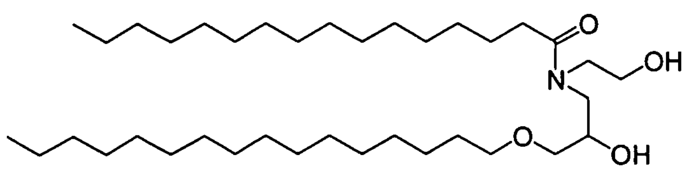

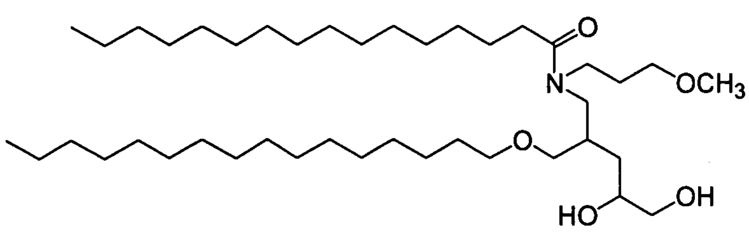

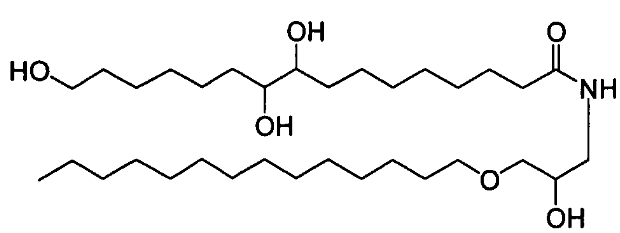

(A-3) Diamide compounds represented by the following formula (3):

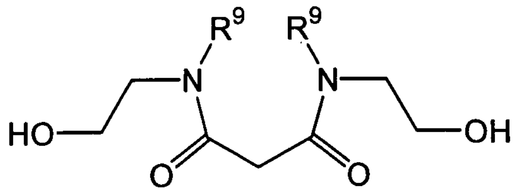

(3)

wherein, $R^9$ represents a $C_{10-18}$ alkyl group which may be substituted with hydroxy group(s).

Specific examples of compound (3) include the compound represented by the following formula:

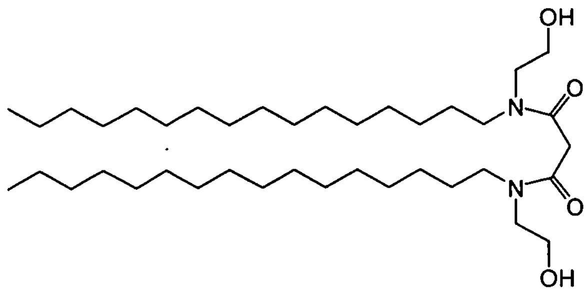

(A-4) Amide compounds represented by the following formula (4):

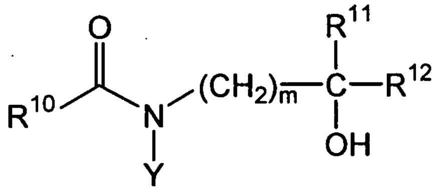

(4)

wherein, $R^{10}$ represents a linear or branched, saturated or unsaturated $C_{9-31}$ hydrocarbon group which may be substituted with hydroxy group(s), or a 2-dodecen-1-yl succinic acid residue, m stands for an integer of from 1 to 3, $R^{11}$ and $R^{12}$ each represents a hydrogen atom or a $C_{1-4}$ alkyl or hydroxyalkyl group, Y represents a linear or branched, saturated or unsaturated $C_{10-32}$ hydrocarbon group which may be substituted with hydroxy group(s), or a substituent represented by the following formula:

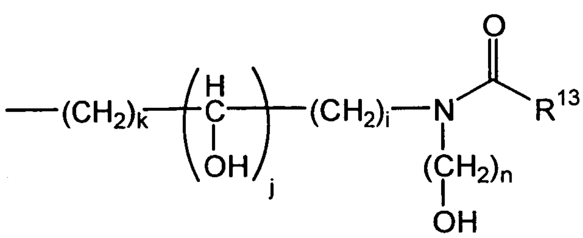

in which, k, i and n each stands for an integer of from 1 to 3, j stands for 0 or 1, and $R^{13}$ represents a linear or branched, saturated or unsaturated $C_{9-31}$ hydrocarbon group which may be substituted with hydroxy group(s).

Specific examples of Compound (4) include a compound represented by the following formula:

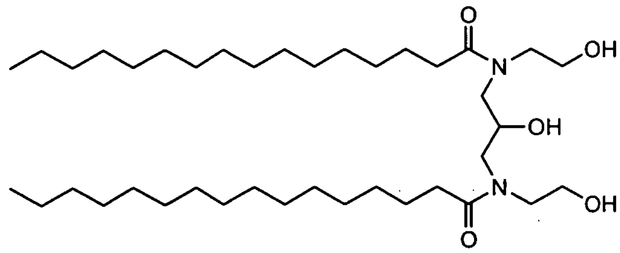

Of the above-described amphipathic amide lipids, those represented by formula (1) or (2) are preferred, those represented by formula (1) or (2b) are more preferred, and those represented by formula (1) are even more preferred.

As Component (A), two or more of these amphipathic amide lipids may be used in combination. Its (their) content in the hair cleansing composition of the present invention is preferably from 0.001 to 20 wt.%, more preferably from 0.1 to 20 wt.%, even more preferably from 0.5 to 15 wt.% in view of imparting suppleness and body to hair and preventing split ends or breakage of hair.

In the quaternary ammonium salt (B) represented by the formula (b-1), R represents an aliphatic hydrocarbon group having 12 to 28 carbon atoms, of which the number of carbon atoms is preferably 16 to 24, more preferably 22. As R, a linear or branched alkyl or alkenyl group is preferred, with a linear alkyl group being more preferred. R' represents a $C_{1-3}$ alkyl group, preferably a methyl group. In general, the length of the carbon chain of a compound to be incorporated in cosmetics varies depending on the raw materials used for the synthesis of the compound. The distribution of the number of carbon atoms of R in the quaternary ammonium salt (b-1) is preferably narrower and the content of the most abundant compound in the quaternary ammonium salt (b-1) is preferably from 70 to 100 wt.%, more preferably from 80 to 100 wt.%. Examples of the anion $X^-$ include halide ions such as chloride ion, bromide ion and the like, and organic anions and the like such as methosulfate ion, ethosulfate ion, methophosphate ion, ethophosphate ion and methocarbonate ion and the like. Of these, halide ions are preferred, with chloride ion being more preferred.

Specific examples of the preferred quaternary ammoniums salt (b-1) include cetyltrimethylammonium chloride, stearyltrimethylammonium chloride, arachyltrimethylammonium chloride and behenyltrimethylammonium chloride and the like. Of these, behenyltrimethylammonium chloride is even more preferred from the viewpoints of feeling of the hair upon lubrication and smoothness of the hair upon rinsing.

With regards to the tertiary amine type compound (B) represented by formula (b-2), when A is other than a hydrogen atom, A is preferably an amide group or hydrocarbon oxy group having 14 to 22, more preferably 18 to 22 carbon atoms in total. Moreover, the hydrocarbon moiety of the group is preferably saturated, more preferably saturated and linear. In this case, B preferably represents a trimethylene group. When A is a hydrogen atom, B preferably represents a $C_{18-22}$ group, of which a saturated group, moreover, a saturated and linear group is preferred. Examples of $Y^1$ or $Y^2$ include methyl, ethyl, propyl, isopropyl, butyl and tert-butyl groups and the like, of which methyl and ethyl groups are preferred, with methyl being more preferred. Specific examples of the preferred tertiary amine type compound (b-2) include N,N-dimethyloctadecyloxypropylamine and stearamidopropyldimethylamine.

The salt of the tertiary amine type compound (b-2) is formed by neutralizing the tertiary amine type compound with an acidic amino acid, organic acid or inorganic acid. Examples of the acidic amino acid include glutamic acid, aspartic acid and the like. Examples of the organic acid include carboxylic acids such as monocarboxylic acids, dicarboxylic acids, hydroxycarboxylic acids and polycarboxylic acids, alkylsulfuric acids, alkylphosphoric acids and the like. As the carboxylic acids, dicarboxylic acids and hydroxycarboxylic acids are preferred. The dicarboxylic acids include malonic acid, succinic acid, glutaric acid, adipic acid, maleic acid, fumaric acid, phthalic acid and the like, while the hydroxycarboxylic acids include glycolic acid, lactic acid, hydroxyacrylic acid, oxybutyric acid, glyceric acid, malic acid, tartaric acid, citric acid and the like. The inorganic acids include phosphoric acid, sulfuric acid, nitric acid and hydrochloric acid. Of these, acidic amino acids and organic acids are preferred, of which glutamic acid, $\alpha$-hydroxycarboxylic acids (suitably, lactic acid and malic acid) are preferred.

As Component (B), two or more compounds may be used in combination. When two or more of the quaternary ammonium salts (b-1) are used in combination, it is preferred that they satisfy the condition described above (the content of the most abundant compound is preferably from 70 to 100 wt.%) as the whole quaternary ammonium salt (b-1). The amount of Component (B) to be used is preferably from 0.1 to 10 wt.%, more preferably from 0.3 to 5 wt.% in the intended hair cosmetic composition.

In the higher alcohol (C) represented by formula (c), R" represents an aliphatic hydrocarbon group having 12 to 28 carbon atoms, of which the number of carbon atoms is preferably 16 to 24, more preferably 22. As R", a linear or branched alkyl or alkenyl group is preferred, with a linear alkyl group being more preferred. Similar to the case of quaternary ammonium salt (b-1), the distribution of the number of carbon atoms of R" in Component (C) is preferably narrower and the content of the most abundant compound in Component (C) is preferably from 70 to 100 wt.%, more

preferably from 80 to 100 wt.%.

The specific examples of the compound preferred as Component (C) include cetyl alcohol, stearyl alcohol, arachyl alcohol, behenyl alcohol, 2-octyllauryl alcohol, 2-hexyldecyl alcohol, isostearyl alcohol, and carnaubyl alcohol (tetracosanol), of which behenyl alcohol is preferred.

As Component (C), two or more compounds may be used in combination. Upon combined use, it is preferred that component (C) as a whole satisfies the condition described above (the amount of the most abundant compound is preferably from 70 to 100 wt.%). The amount of Component (C) is preferably from 0.1 to 50 wt.%, more preferably from 0.5 to 20 wt.%, still more preferably from 1 to 10 wt.% in the intended hair cosmetic composition.

In the present invention, when the quaternary ammonium salt (b-1) is contained as Component (B), it is preferred that R and R" of the most abundant compounds in the quaternary ammonium salt (b-1) and Component (C), respectively, have an equal number of carbon atoms. In addition, R and R" each preferably represents a linear alkyl group from the viewpoints of excellent lubrication and flexible feeling during from application of the composition to the hair to rinsing. The alkyl group has preferably 16 to 24, more preferably 22 carbon atoms.

The hair cosmetic composition of the present invention may further contain a cationic polymer in consideration of the texture of the foam formed when using the hair cosmetic composition, feeling of such foam on lubrication, reduction in the friction between hair strands upon washing and smoothness after drying. Examples of the cationic polymer include cationic cellulose derivatives, cationic starch, cationic guar gum derivatives, homopolymers of a diallyl quaternary ammonium salt, diallyl quaternary ammonium salt/acrylamide copolymers, quaternized polyvinylpyrrolidone derivatives, polyglycol-polyamine condensates, vinylimidazolium trichloride/vinylpyrrolidone copolymers, hydroxyethyl cellulose/dimethyldiallyl ammonium chloride copolymers, vinylpyrrolidone/quaternized dimethylaminoethyl methacrylate copolymers, polyvinylpyrrolidone/alkylamino acrylate copolymers, polyvinylpyrrolidone/alkylaminoacrylate/vinyl caprolactam copolymers, vinylpyrrolidone/methacrylamidopropyl trimethylammonium chloride copolymers, alkylacrylamide/acrylate/alkylaminoalkyl acrylamide/polyethylene glycol methacrylate copolymers, adipic acid/dimethylaminohydroxypropylethylenetriamine copolymers (CALTALETINE, product of US Sandos Corp.), and cationic polymers described in Japanese Patent Laid Open No. Sho 53-139734 and Japanese Patent Laid Open No. Sho 60-36407. Of these, cationic cellulose derivatives and cationic guar gum derivatives are preferred.

Two or more of these cationic polymers may be used in combination. The content of the cationic polymers in the hair cosmetic composition of the present invention is preferably from 0.02 to 5 wt.%, more preferably from 0.05 to 1 wt.%, and even more preferably from 0.1 to 0.3 wt.% from the viewpoints of improvement in the foam quality upon washing, manageability of hair after drying and improvement in hair feel.

The hair cosmetic composition of the present invention may further contain a conditioning component such as silicone and the like in order to improve the finish after drying. Examples of such include dimethylpolysiloxane, methylphenylpolysiloxane, amino-modified silicone, polyether-modified silicone, epoxy-modified silicone, fluorine-modified silicone, cyclic silicones, alkyl-modified silicones, and oxazoline-modified silicone and the like. Of these, dimethylpolysiloxane, methylphenylpolysiloxane, amino-modified silicone, polyether-modified silicone, oxazoline-modified silicone and cyclic silicones are preferred. Two or more of these silicones may be used in combination. The content of the conditioning component preferably ranges from 0.01 to 20 wt.%, more preferably from 0.05 to 10 wt.%, still more preferably from 0.1 to 5 wt.% in the intended hair cosmetic composition.

The hair shampoo composition of the present invention may contain, in addition to the components described above, oil components such as cholesterol and derivatives thereof, petrolatum, lanolin derivatives, and fatty acid esters of polyethylene glycol; polymer emulsions such as polycarboxylic acid, cross-linked carboxylic acid/carboxylate ester copolymers, crosslinked acrylic acid/acrylate ester copolymers and acrylamide/acrylamide butanesulfonate copolymer; polyhydric alcohols such as glycerin and sorbitol; humectants; chelating agents such as ethylene diamine tetraacetic acid (EDTA); drugs such as vitamins; amino acids and derivatives thereof; fine powder of a polymer such as polyethylene, polystyrene, polymethylmethacrylate, nylon or silicone and the like, and hydrophobic products thereof; extracts derived from animals or plants; ultraviolet absorbent; pearling agents, antiseptics; bactericides; anti-inflammatories; antidandruffs; pH regulators; colorants; and perfumes, according to the intended purpose.

In the present invention, a hair cosmetic composition is prepared by emulsifying an aqueous composition containing Components (A), (B) and (C) at a temperature equal to or greater than the melting point of Component (C) but less than the phase transition temperature of a three-component composition composed of Component (B) and Component (C), each added in amounts equal to the contents thereof in the intended hair cosmetic composition, respectively, and the balance water. By this process, a hair cosmetic composition capable of imparting excellent lubrication and flexibility to the hair during from application to washing rinsing, and having high emulsion stability can be obtained.

The emulsification upon preparation of the three-component composition to be used for the measurement of the phase transition temperature is preferably performed at about 90°C. When two or more compounds are employed for Component (B) or (C), a three-component composition having all of them emulsified in water is employed. When two or more compounds are used as Component (C), the melting point of the higher alcohol most abundantly contained

can be used as an approximate melting point of Component (C). When two or more higher alcohols are contained as Component (C) and the amount of those are equal to each other and greatest in the component, the melting point of the higher alcohol whose melting point is higher is preferably used. The phase transition temperature and melting point can be measured by a differential scanning calorimeter (for example, "DSC120" of SSC5200 series, product of SEIKO) at a heating rate of 1 °C/min. The composition provided for the measurement of the phase transition temperature and the hair cosmetic composition whose emulsifying temperature is set based on the phase transition temperature and melting point thus measured are each preferably emulsified under normal pressure by stirring with a propeller, stirring in a homomixer or mixing in a static mixer. Mixing while stirring is preferably continued until a substantially complete dissolution of imperfectly dispersed substances such as crystals of Component (C) is achieved.

The hair cosmetic composition according to the present invention can be prepared in the liquid form, gel form and the like, of which the liquid form is preferred.

The hair cosmetic composition according to the present invention is preferably prepared as ones to be used in the shower/bath room such as a 2 in 1 shampoo-and-conditioner, treatment and conditioner, of which conditioner is more preferred.

-EXAMPLES-

The present invention will hereinafter be described more specifically by Examples but will not be limited to or by them.

The amphipathic amide lipid employed in the Examples and Comparative Examples is represented by the following compounds.

Amphipathic amide lipid A

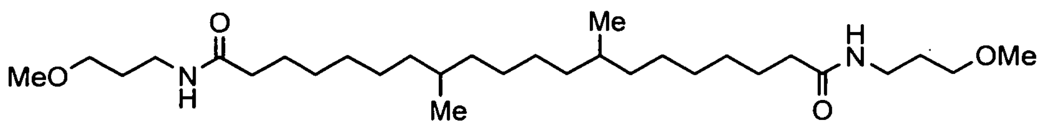

Amphipathic amide lipid B

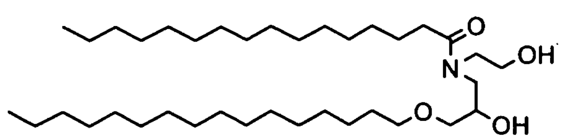

Examples 1 to 5, Comparative Examples 1 to 3

The hair conditioners as shown in Table 1 were prepared and evaluated.

(Preparation Process)

(1) In a 500-mL beaker, purified water and necessary amounts of components other than methylparaben were charged to give the final amount of 400 g, followed by heating to 90°C. The reaction mixture was stirred by a three-bladed propeller at 250 rpm.

(2) Methylparaben was dissolved in heated purified water, to which the mixture obtained in (1) was added, followed by emulsification. The melting point of behenyl alcohol and the gel phase transition temperature of a composition obtained by emulsifying 1.5 wt.% of behenyltrimethylammonium chloride and 5 wt.% of behenyl alcohol in water were measured by a differential scanning calorimeter ("DSC120" of SSC5200 series, product of SEIKO, at heating

rate of 1 °C/min). The result showed the melting point of behenyl alcohol was at about 72°C and the phase transition temperature was at about 85°C. The emulsifying temperature was therefore set at about 75°C.
(3) After cooling to 45°C, a perfume was added and the mixture was cooled further to 40°C while stirring, whereby the hair conditioner of Example 1 was obtained.

In accordance with the process described above, hair conditioners of Examples 2 to 5, and Comparative Examples 2 and 3 were prepared. In Comparative Example 1, the emulsifying temperature was set at 88°C, higher than the gel phase transition temperature, which was at 85°C, of a composition obtained by emulsifying 1.5 wt.% of stearyltrimethylammonium chloride and 5 wt.% of behenyl alcohol in water.

(Organoleptic evaluation)

After hair was shampooed with a commercially available cleansing type shampoo, 2 g of the hair conditioner was applied and the lubrication of the hair upon application was evaluated. The hair was then rinsed with running water at 40°C at 4 L/min and smoothness upon rinsing was then evaluated, followed by towel drying. The hair was blow-dried, and the evaluation was done thereafter by a panel of 5 experts based on the criteria described below, and the total score of which is shown.
(1) Feeling upon Lubrication
 4: The hair has natural and good lubrication.
 3: The hair has lubrication.
 2: Difficult to judge whether the hair has lubrication or not.
 1: Slight friction exists between hair strands.
 0: Friction exists between hair strands.
(2) Smoothness upon rinsing
 4: The hair has natural and good smoothness.
 3: The hair has smoothness.
 2: Difficult to judge whether the hair has smoothness or not.
 1: Slight friction exists between hair strands.
 0: Friction exists between hair strands.
(3) Resilience and body of the hair after drying
 4: A marked improvement in resilience and body is observed.
 3: An improvement in resilience and body is observed.
 2: A slight improvement in resilience and body is observed.
 1: Neither resilience nor body is improved.
 0: The hair has lost resilience and body.
(4) Effect-remaining feel
 4: Marked effect-remaining feel exists.
 3: Effect-remaining feel exists.
 2: Difficult to judge whether effect-remaining feel exists or not.
 1: Slight effect-remaining feel exists.
 0: No effect-remaining feel exists.

(Emulsion stability)

The composition was stored in a clear glass container (100 mL) at 50°C for 1 month and a change in appearance was observed.
A: No change occurred.
B: A Slight change occurred.
C: Separation or gelation has occurred.

(Strength of hair)

The percent of undamaged hair by brushing was measured using a split-end generator. A tress (100 hairs, 0.1 g) made of non-chemically-treated hairs (Japanese female, 3 years old) was used for evaluation. The tress was bundled by an acrylic plate (15 x 10 mm) disposed above and the length of the tress therefrom to the end of the hair was adjusted to be 15 cm and the length including a hook (wire) to attach the hair to the split-end generator was adjusted to 16 cm. This tress was treated with each conditioner and then brushed.
Brushing was carried out using a rotary brush engaged with a motor under the conditions of a brush rotation

speed of 100 rpm, 25 to 27°C and 21 to 25%RH (environment adjustable chamber). Brushing was conducted for 90 minutes, with 30 minutes as one unit. When the brushing was conducted for 90 minutes, the state of the hair end (not damaged/discolored/broken/split ends) were observed and the number of each of them was counted. Each measurement was conducted at n=2 or 3 and an average of the values determined in accordance with the following equation is designated as the "percent of undamaged hair" as defined below.

$$\text{The percent of undamaged hair (\%)} = [(\text{total number of hairs}) - (\text{the number of discolored hairs} + \text{broken hairs} + \text{hairs with split ends})] / (\text{total number of hairs}) \times 100$$

Table 1

| (wt.%) | | | | | | | | |
|---|---|---|---|---|---|---|---|---|
| | | Examples | | | | | Comparative Examples | |
| | | 1 | 2 | 3 | 4 | 5 | 1 | 2 |
| (A) | Amphipathic amide lipid A | 2 | 2 | - | 2 | - | 2 | - |
| | Amphipathic amide lipid B | - | - | 2 | - | 2 | - | - |
| (B) | Behenyltrimethylammonium chloride (Purity > 98 wt.%) | 1.5 | 1.2 | 0.2 | - | - | 0.2 | 1.2 |
| | Stearyltrimethylammonium chloride (purity >97 wt.%) | - | 0.2 | 1.2 | - | - | 1.2 | 0.2 |
| | Cetyltrimethylammonium chloride (purity > 98 wt.%) | - | 0.1 | 0.1 | - | - | 0.1 | 0.1 |
| | N,N-Dimethyloctadecyloxypropylamine | - | - | - | 1.5 | - | - | - |
| | Stearamidopropyldimethylamine | - | - | - | - | 1.5 | - | - |
| (C) | Behenyl alcohol (purity > 95 wt.%, melting point: 72°C) | 5 | 4.25 | 0.5 | 4 | 0.5 | 0.5 | 4.25 |
| | Stearyl alcohol (purity > 99 wt.%, melting point: 59°C) | - | 0.6 | 4 | 0.5 | 4 | 4 | 0.6 |
| | Cetyl alcohol (purity > 98 wt.%, melting point: 50°C) | - | 0.15 | 0.5 | - | - | 0.5 | 0.15 |
| Others | Dimethylpolysiloxane emulsion *1 | 2 | - | - | - | 2 | - | 2 |
| | Amino-modified silicone *2 | - | 0.5 | 0.5 | 0.5 | - | 0.5 | - |
| | Dipropylene glycol | 0.5 | 0.5 | 0.5 | 0.5 | 0.5 | 0.5 | 0.5 |
| | Dimethicone | 1 | 1 | 1 | 1 | 1 | 1 | 1 |
| | Benzyloxyethanol | 0.3 | 0.3 | 0.3 | 0.3 | 0.3 | 0.3 | 0.3 |
| | Phenoxyethanol | 0.5 | 0.5 | 0.5 | 0.5 | 0.5 | 0.5 | 0.5 |
| | 50 wt.% NaOH aq. soln./ 50 wt.% citric acid aq. soln. | q.s. *3 | q.s. *3 | q.s. *3 | q.s. *3 | q.s. *3 | q.s. *3 | q.s. *3 |
| | Purified water | Balance | Balance | Balance | Balance | Balance | Balance | Balance |
| pH | | 3.5 | 3.5 | 3.5 | 3.5 | 3.5 | 3.5 | 3.5 |
| Buffer capacity (NaOH-gram equivalent weight/L) | | 0.01 | 0.01 | 0.01 | 0.01 | 0.01 | 0.01 | 0.01 |

*1: Silicone CF-2460 (product of Dow Corning Toray Silicone)

*2: Silicone SM8704C (product of Dow Corning Toray Silicone)

*3: Amount to adjust pH

Table 1 (continued)

| (wt.%) | | | | | | | | |
|---|---|---|---|---|---|---|---|---|
| | | Examples | | | | | Comparative Examples | |
| | | 1 | 2 | 3 | 4 | 5 | 1 | 2 |
| Phase transition temperature of hair cosmetic composition (°C) | | 88 | 85 | 75 | 85 | 85 | 75 | 85 |
| Heating temperature for emulsification upon preparation (°C) | | 75 | 75 | 65 | 75 | 75 | 80 | 75 |
| Evaluation | Lubrication upon application | 20 | 18 | 15 | 19 | 18 | 5 | 8 |
| | Smoothness upon rinsing | 19 | 20 | 14 | 19 | 20 | 7 | 11 |
| | Resilience and body of the hair | 20 | 19 | 17 | 18 | 19 | 6 | 12 |
| | Effect remaining feel | 19 | 18 | 19 | 18 | 18 | 8 | 6 |
| | Emulsion stability | A | A | A | A | A | C | A |
| | Strength of hair (percent of undamaged hair) | 100 | 96 | 98 | 96 | 96 | 50 | 65 |

Example 6: Hair treatment

A composition obtained by emulsifying 2.4 wt.% of N,N-dimethyloctadecyloxypropylamine and 7.2 wt.% of stearyl alcohol in water has a phase transition temperature of 80°C and the melting point of stearyl alcohol is 59°C. The hair treatment described below was therefore prepared in accordance with Example 1 by setting the emulsifying temperature at 62°C.

| | (wt.%) |
|---|---|
| N,N-Dimethyloctadecyloxypropylamine | 2.4 |
| Stearyl alcohol | 7.2 |
| Propylene glycol | 2.0 |
| Amphipathic amide lipid A | 0.5 |
| Sunflower oil | 0.9 |
| Camellia oil | 0.2 |
| Cholesteryl hydroxystearate | 0.1 |
| Dipentaerythritol fatty acid ester *1 | 0.3 |
| Benzyloxyethanol | 0.5 |
| Methylpolysiloxane (100000 mPa·s) | 3.7 |
| Aqueous solution of lactic acid (90 wt.%) | 0.9 |
| Methylparaben | 0.1 |
| Perfume | 0.3 |
| Purified water | Balance |

*1: Hydroxystearic acid, stearic acid and rosin acid constitute the fatty acid.

The hair treatment described above can provide benefits such as good smoothness and moisturized feel to the shampooed hair and prevent appearance of split ends or broken hair.

Example 7: Hair treatment

A composition obtained by emulsifying 3.1 wt.% of stearamidopropyldimethylamine and 9.0 wt.% of stearyl alcohol in water has a phase transition temperature of 80°C and the melting point of stearyl alcohol is 59°C. The hair treatment described below was therefore prepared in accordance with Example 1 by setting the emulsifying temperature at 65°C.

| | (wt.%) |
|---|---|
| Stearamidopropyldimethylamine | 3.1 |
| Stearyl alcohol | 9.0 |
| Hydroxyethylcellulose | 0.3 |
| Dipropylene glycol | 2.5 |
| Amphipathic amide lipid A | 0.05 |
| Liquid paraffin | 0.5 |
| Benzyloxyethanol | 1.5 |
| Phenoxyethanol | 0.1 |
| Methylpolysiloxane (100000 mPa·s) | 3.0 |
| Amino-modified silicone | 0.75 |
| Aqueous solution of lactic acid (90 wt.%) | 0.9 |
| Aqueous solution of malic acid (50 wt.%) | 0.02 |
| Perfume | 0.4 |
| Purified water | Balance |

The hair treatment described above can provide benefits such as good smoothness and moisturized feel to the shampooed hair and prevent appearance of split ends or broken hair.

Example 8: Hair conditioner

A composition obtained by emulsifying 3.4 wt.% of stearamidopropyldimethylamine and 9.0 wt.% of stearyl alcohol in water has a phase transition temperature of 80°C and the melting point of stearyl alcohol is 59°C. The hair conditioner described below was therefore prepared in accordance with Example 1 by setting the emulsifying temperature at 62°C.

| | (wt.%) |
|---|---|
| Stearamidopropyldimethylamine | 3.4 |
| Stearyl alcohol | 9.0 |
| Glutamic acid | 1.5 |
| Benzyloxyethanol | 1.5 |
| Dipropylene glycol | 2.5 |
| Phenoxyethanol | 0.1 |
| Amphipathic amide lipid B | 0.5 |
| Aqueous solution of malic acid (50 wt.%) | 0.02 |
| Dipentaerythritol fatty acid ester | 0.2 |
| Methylpolysiloxane mixture | 2.5 |
| Highly polymerized methylpolysiloxane ·decamethylcyclopentasiloxane mixture | 2.5 |
| Aminoethylaminopropylsiloxane·dimethylsiloxane copolymer emulsion | 2.5 |
| Hydroxyethyl cellulose | 0.3 |
| Liquid paraffin | 0.5 |
| Sodium hydroxide | Amount to adjust pH |
| Perfume | 0.5 |
| Purified water | Balance |

The hair conditioner described above can provide benefits such as good smoothness and moisturized feel to the shampooed hair and prevent appearance of split ends or broken hair.

## Claims

1. A process for preparing a hair cosmetic composition comprising the following components (A), (B) and (C):

    (A) an amphipathic amide lipid;
    (B) a quaternary ammonium salt represented by the following formula (b-1):

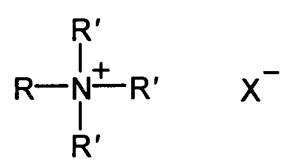

(b-1)

wherein, R represents a $C_{12-28}$ aliphatic hydrocarbon group, R' represents a $C_{1-3}$ alkyl group and $X^-$ represents an anion; or a tertiary amine type compound represented by the following formula (b-2):

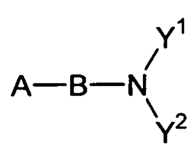

(b-2)

wherein, A represents a hydrogen atom or a linear or branched, and saturated or unsaturated amide, N-hydrocarbon carbamoyl group, acyloxy group or hydrocarbon oxy group having 12 to 24 carbon atoms in total, B represents a linear or branched, saturated or unsaturated divalent $C_{1-22}$ hydrocarbon group, $Y^1$ and $Y^2$ each independently represents a $C_{1-4}$ alkyl group or salt thereof; and
(C) a higher alcohol represented by the following formula (c):

$$R''\text{-}OH \qquad (c)$$

wherein, R" represents an aliphatic $C_{12-28}$ hydrocarbon group; wherein the process comprises the step of emulsifying an aqueous composition containing components (A), (B) and (C) at a temperature equal to or greater than the melting point of component (C) but less than the phase transition temperature of a three-component composition comprising component (B) and component (C), each added in amounts equal to the contents thereof in the intended hair cosmetic composition, respectively, and the balance water.

2. The process of Claim 1, wherein component (B) contains the quaternary ammonium salt represented by formula (b-1), wherein the number of carbon atoms of R of the most abundant compound in the quaternary ammonium salt (b-1) is equal to that of R" of the most abundant compound in component (C), and said compounds in Components (B) and (C) are, respectively, each contained in amount of from 70 to 100 wt.%.

3. The process of Claim 2, wherein the number of carbon atoms which is equal between said two compounds is 22.

4. The process of any one of Claims 1 to 3, wherein component (A) is selected from the group consisting of amphipathic amide lipids represented by any one of the following formulas (1) to (4):

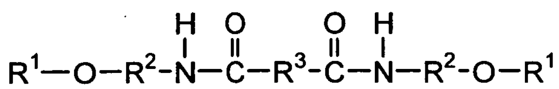
(1)

wherein, $R^1$ represents a linear or branched $C_{1-12}$ hydrocarbon group which may be substituted with hydroxyl group(s) and/or alkoxy group(s), $R^2$ represents a linear or branched divalent $C_{1-5}$ hydrocarbon group, and $R^3$ represents a linear or branched divalent $C_{1-22}$ hydrocarbon group;

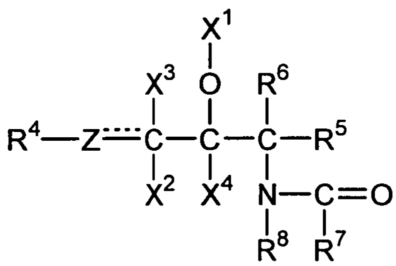
(2)

wherein, $R^4$ represents a linear, branched or cyclic, saturated or unsaturated $C_{4-30}$ hydrocarbon group which may be substituted with hydroxyl, oxo or amino group(s), Z represents a methylene group, a methine group or an oxygen atom, a broken line represents the presence or absence of a n bond, $X^1$ represents a hydrogen atom, an acetyl group or a glyceryl group, or, forms an oxo group together with the adjacent oxygen atom, $X^2$, $X^3$ and $X^4$ each independently represents a hydrogen atom, a hydroxyl group or an acetoxy group, wherein when Z represents a methine group, one of $X^2$ and $X^3$ represents a hydrogen atom and the other does not exist, and when -O-$X^1$ represents an oxo group, $X^4$ does not exist, $R^5$ and $R^6$ each independently represent a hydrogen atom, a hydroxyl group, a hydroxymethyl group or an acetoxymethyl group, $R^7$ represents a linear, branched or cyclic, saturated $C_{5-35}$ hydrocarbon group which may be substituted with a hydroxyl or amino group, or the saturated $C_{5-35}$ hydrocarbon group in which a linear, branched or cyclic, saturated or unsaturated $C_{8-22}$ fatty acid which may be substituted with hydroxyl group(s) is ester-bonded to the ω-position of the hydrocarbon group, and $R^8$ represents a hydrogen atom or a linear or branched, saturated or unsaturated hydrocarbon group which may have substituent (s) selected from a hydroxyl group, hydroxyalkoxy groups, alkoxy groups and an acetoxy group and has from 1 to 8 carbon atoms in total;

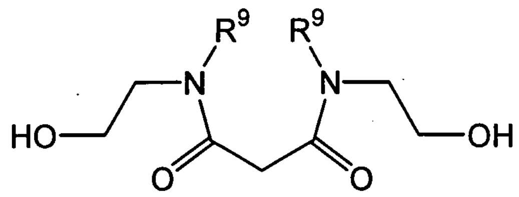

(3)

$R^9$ represents a $C_{10-18}$ alkyl group which may be substituted with hydroxyl group(s);

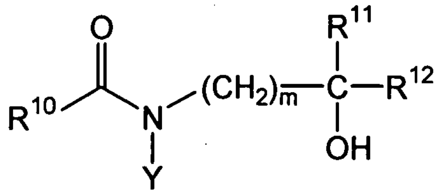

(4)

wherein, $R^{10}$ represents a linear or branched, saturated or unsaturated $C_{9-31}$ hydrocarbon group which may be substituted with hydroxyl group(s), or a 2-dodecen-1-yl succinic acid residue, m stands for an integer of from 1 to 3, $R^{11}$ and $R^{12}$ each represents a hydrogen atom or $C_{1-4}$ alkyl or hydroxyalkyl group, Y represents a linear or branched, saturated or unsaturated $C_{10-32}$ hydrocarbon group which may be substituted with hydroxyl group(s), or a substituent represented by the following formula:

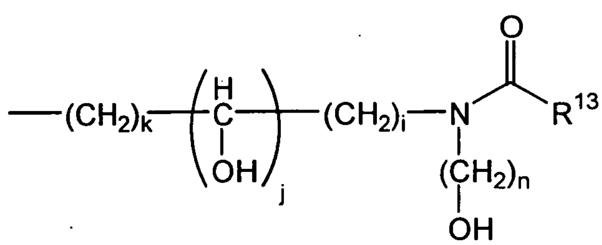

in which, k, i and n each stands for an integer of from 1 to 3, j stands for 0 or 1, and $R^{13}$ represents a linear or branched, saturated or unsaturated $C_{9-31}$ hydrocarbon group which may be substituted with hydroxyl group(s); and mixtures thereof.

**5.** The process of Claim 4, wherein component (A) is selected from amphipathic amide lipids represented by formula (1) or formula (2).

**6.** A hair cosmetic composition comprising the following components (A), (B) and (C):

(A) an amphipathic amide lipid,
(B) a quaternary ammonium salt represented by the following formula (b-1):

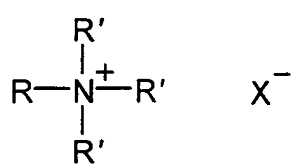

(b-1)

wherein, R represents a $C_{12-28}$ aliphatic hydrocarbon group, R' represents a $C_{1-3}$ alkyl group and $X^-$ represents an anion; or a tertiary amine type compound represented by the following formula (b-2):

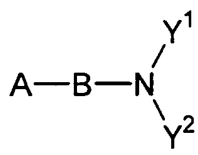

(b-2)

wherein, A represents a hydrogen atom or a linear or branched, and saturated or unsaturated amide, N-hydrocarbon carbamoyl group, acyloxy group or hydrocarbon oxy group having 12 to 24 carbon atoms in total, B represents a linear or branched, saturated or unsaturated divalent $C_{1-22}$ hydrocarbon group, $Y^1$ and $Y^2$ each independently represents a $C_{1-4}$ alkyl group or salt thereof; and

(C) a higher alcohol represented by the following formula (c):

**R"-OH** (c)

wherein, R" represents an aliphatic $C_{12-28}$ hydrocarbon group,
wherein such composition is obtainable by emulsifying an aqueous composition containing components (A), (B) and (C) at a temperature equal to or greater than the melting point of component (C) but less than the phase transition temperature of a three-component composition comprising component (B) and component (C), each added in amounts equal to the contents thereof in the intended hair cosmetic composition, respectively, and the balance water.

European Patent Office

# EUROPEAN SEARCH REPORT

Application Number
EP 04 01 4468

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 221 371 B1 (BAIK IN-SUB ET AL) 24 April 2001 (2001-04-24) * example 17 * ----- | 1,6 | A61K7/075 |
| X | US 6 110 450 A (BERGMANN WOLFGANG ROBERT) 29 August 2000 (2000-08-29) * example 2 * ----- | 1,4-6 | |
| X | WO 01/24767 A (BONE ERIC ; OREAL (FR); YAMADA SHINICHI (JP)) 12 April 2001 (2001-04-12) * page 12, line 27 - page 13, line 26 * ----- | 1,4-6 | |
| X | EP 1 153 595 A (TAKASAGO PERFUMERY CO LTD) 14 November 2001 (2001-11-14) * paragraph [0060]; examples 14,15 * ----- | 1,4-6 | |
| X | EP 1 167 374 A (KITASATO INST ; KIBUN FOOD CHEMIFA (JP)) 2 January 2002 (2002-01-02) * example 18 * ----- | 1,6 | |
| | | | TECHNICAL FIELDS SEARCHED (Int.Cl.7) |
| X,D | EP 1 174 111 A (KAO CORP) 23 January 2002 (2002-01-23) * paragraphs [0006], [0019]; example 4 * ----- | 1-3,6 | A61K |
| A | DE 299 01 069 U (GOLDWELL GMBH) 21 June 2000 (2000-06-21) ----- | | |
| A | EP 1 166 766 A (KAO CORP) 2 January 2002 (2002-01-02) ----- | | |
| A | EP 1 283 030 A (KAO CORP) 12 February 2003 (2003-02-12) ----- | | |

The present search report has been drawn up for all claims

| Place of search | Date of completion of the search | Examiner |
|---|---|---|
| Munich | 24 September 2004 | Sala-Jung, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 01 4468

This annex lists the patent family members relating to the patent documents cited in the above-mentioned European search report. *The members are as contained in the European Patent Office EDP file on*
The European Patent Office is in no way liable for these particulars which are merely given for the purpose of information.

24-09-2004

| Patent document cited in search report | | Publication date | Patent family member(s) | | Publication date |
|---|---|---|---|---|---|
| US 6221371 | B1 | 24-04-2001 | JP 2001509138 | T | 10-07-2001 |
| | | | WO 9821176 | A1 | 22-05-1998 |
| | | | KR 2000052640 | A | 25-08-2000 |
| US 6110450 | A | 29-08-2000 | AU 5976799 | A | 03-04-2000 |
| | | | WO 0015181 | A1 | 23-03-2000 |
| WO 0124767 | A | 12-04-2001 | JP 2001114629 | A | 24-04-2001 |
| | | | AU 7669800 | A | 10-05-2001 |
| | | | EP 1135095 | A1 | 26-09-2001 |
| | | | WO 0124767 | A1 | 12-04-2001 |
| EP 1153595 | A | 14-11-2001 | JP 2001316217 | A | 13-11-2001 |
| | | | EP 1153595 | A2 | 14-11-2001 |
| | | | US 2002010215 | A1 | 24-01-2002 |
| EP 1167374 | A | 02-01-2002 | JP 2002010797 | A | 15-01-2002 |
| | | | CA 2351836 | A1 | 29-12-2001 |
| | | | DE 60100773 | D1 | 23-10-2003 |
| | | | DE 60100773 | T2 | 15-07-2004 |
| | | | EP 1167374 | A1 | 02-01-2002 |
| | | | US 2002037291 | A1 | 28-03-2002 |
| EP 1174111 | A | 23-01-2002 | JP 2002029937 | A | 29-01-2002 |
| | | | CN 1334073 | A | 06-02-2002 |
| | | | EP 1174111 | A2 | 23-01-2002 |
| | | | US 2003147839 | A1 | 07-08-2003 |
| | | | US 2002025302 | A1 | 28-02-2002 |
| DE 29901069 | U | 21-06-2000 | DE 29901069 | U1 | 21-06-2000 |
| EP 1166766 | A | 02-01-2002 | EP 1166766 | A1 | 02-01-2002 |
| | | | US 6685953 | B1 | 03-02-2004 |
| | | | CN 1347312 | T | 01-05-2002 |
| | | | WO 0061097 | A1 | 19-10-2000 |
| | | | US 2004115162 | A1 | 17-06-2004 |
| EP 1283030 | A | 12-02-2003 | CN 1404813 | A | 26-03-2003 |
| | | | EP 1283030 | A1 | 12-02-2003 |
| | | | JP 2004002261 | A | 08-01-2004 |
| | | | US 2003147822 | A1 | 07-08-2003 |

EPO FORM P0459

For more details about this annex : see Official Journal of the European Patent Office, No. 12/82